# EUROPEAN PATENT SPECIFICATION

(11) **EP 0 299 299 B1**
(45) Date of publication and mention of the grant of the patent: **05.10.1994**
(21) Application number: 88110558.9
(22) Date of filing: 01.07.1988
(51) Int. Cl.: G01N 33/543

(54) **Diagnostic system employing a unitary substrate to immobilize microspheres**
Diagnostisches System mit Anwendung eines einheitlichen Substrates für die Immobilisierung von Mikrosphären
Système diagnostique utilisant un substrat unitaire pour immobiliser les microsphères

(30) Priority: 17.07.1987 US 74968
(43) Date of publication of application: 18.01.1989
(73) Proprietor: POREX TECHNOLOGIES CORP. OF GEORGIA, Fairburn Georgia 30213 (US)
(72) Inventor: Smith, Michael W., Peachtree City, GA 30269 (US); Pierce, Robert S., Smyrna, GA 30080 (US)
(74) Representative: DIEHL GLAESER HILTL & PARTNER

(56) References cited:
- EP-A- 0 207 721
- WO-A-85/05451
- BE-A- 711 286
- FR-A- 2 502 508
- WPI/DERWENT ABSTRACTS; no. 80-85124C#

## Description

The present invention relates to an improved diagnostic testing system of the type in which microspheres or other small particles are immobilized at or near the top surface thereof.

### BACKGROUND OF THE INVENTION

A number of immunoassay systems have been developed utilizing immunologic technology. In these systems, the presence of an antigen in a test sample is detected by causing the antigen to bind to antibodies in a selected testing medium and then detecting the presence of the antigen in the testing medium. In a recently developed system, the testing procedure and the time to carry out the testing process has been substantially reduced so as to make it convenient to carry out the immunoassay process in a doctor's office or by a patient at home.

This prior art system as shown in Fig. 1 and represented by the disclosure of e.g. WO-A-85/05451, comprises a container 11 housing an absorbent cylinder 13, which serves as a reservoir. On top of the reservoir is a porous plastic disk 15 on which is supported a porous membrane 17 usually separated from the porous plastic disk 15 by a porous contact pad 19. In the immunoassay process, antibodies specific to the antigen being detected are deposited on the membrane 19 by first bonding the antibodies or adsorbing the antibodies on microspheres of a synthetic resin such as polystyrene or latex and then depositing these microspheres in or on the membrane by passing liquid in which the microspheres are suspended through the membrane. A test sample to be tested for the presence of the suspect antigen is poured on the membrane 17 and is drawn through the membrane 17 relatively rapidly by capillary action of the reservoir 13 in combination with the porous plastic disk 15 and the contact pad 19. If the suspect antigen is in the test sample, it will bind to the antibodies deposited on or in the membrane. Following this step, liquid containing labelled antibodies specific to the suspect antigen is poured onto the membrane and is drawn through the membrane into the reservoir 13 by capillary action. If the antigen has been extracted from the test sample and retained in or on the membrane, the labelled antibodies will bind to the antigen and thus remain in or on the membrane 19. The application of the labelled antibody to the membrane will usually be followed by a rinsing step to remove unbound labelled antibody. This rinsing step is then followed by a step of causing the labelled antibody to exhibit its presence. In the case of an enzyme label, this last step is carried out by addition of a solution of a color forming agent which reacts with the enzyme as the solution passes through it. As each liquid is passed through the membrane, it is absorbed by reservoir and the reservoir must be large enough to hold all of the volume of the test specimen, the labelled antibody solution, the rinse solution and the color forming solution.

To obtain an accurate indication of the presence or absence of the antigen, the system must cause an even consistent flow of the various liquids which are passed through the membrane. In order to achieve this even flow, contact between the various components of the system must be maintained. Any gaps between any two of the components will create pools of stagnant liquid and interfere with the capillary action drawing the liquid into the reservoir. This interference leads to erroneous or vague test results. Thus, when assembling the components of the system, great care must be taken to avoid trapping any particulate matter between the components, which particulate matter would create gaps between the components. The material of the membrane is available only as relatively large flat sheets and they must be cut into disks for use in the system. The assembly of the components is difficult because both the membrane and the contact pad are flimsy and manual assembly is usually required.

### SUMMARY OF THE INVENTION

The system of the present invention is designed to overcome the above described problems in the prior art system. In accordance with the present invention, a one piece porous substrate is provided in the container and serves both to extract the target substrate in or on a top layer thereof with the remainder of the pore structure of the substrate serving as a reservoir. The pores in the top surface of the porous substrate are microscopic for entrapment of macroscopic particles such as microspheres. The pores in all but the thin top layer of the substrate have a much greater pore size and this portion of the substrate with the larger pores acts as a reservoir for the liquids to be passed through the top layer.

Because the reservoir and layer in or on which the microspheres are entrapped are of one piece, the problems of assembly and the potential gaps between the components of the prior art system are avoided.

### BRIEF DESCRIPTION OF THE DRAWINGS

Fig. 1 is a sectional view illustrating immunoassay apparatus of the prior art;
Fig. 2 is a sectional view illustrating the diagnostic apparatus of the present invention;
Figs. 3 and 4 are a sectional view of a mold illustrating successive steps in a method of manufacturing of a porous substrate to be used in the apparatus of the present invention;
Fig. 5 illustrates a porous slug which is made by the methods illustrated in Figs. 3 and 4;
Figs. 6 through 8 illustrate sectional views through a mold illustrating the steps of an alternative method of making a porous substrate to be employed in the apparatus of the present invention.

### DESCRIPTION OF A SPECIFIC EMBODIMENT

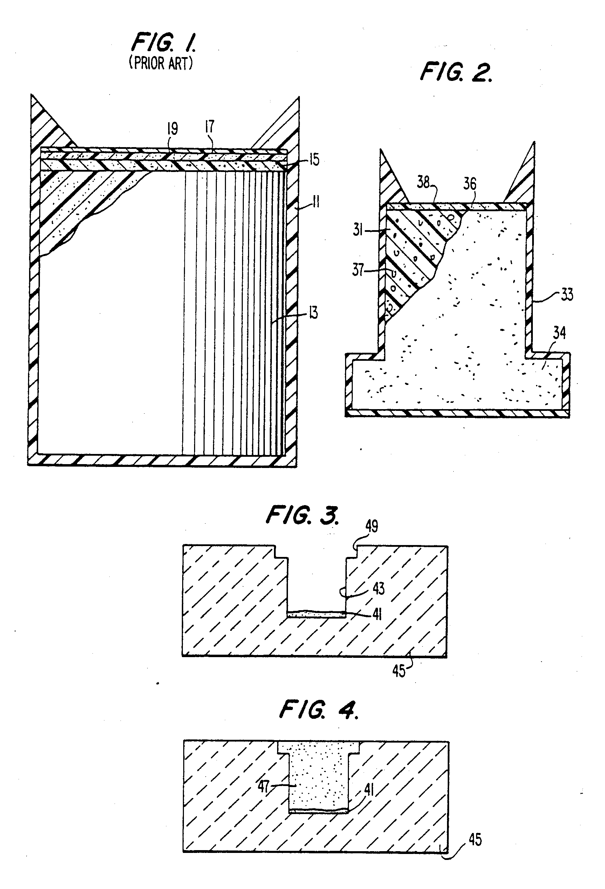

As shown in Fig. 2, the testing device of the present invention comprises a porous substrate 31 made of one piece contained within and filling a plastic container 33. The container may be made of two pieces to facilitate inserting the substrate into the container. The substrate 31 is cylindrical in shape and has an enlarged section 34 at the bottom. The pores of the substrate are interconnected into tortuous capillary passageways so that the substrate is absorbent. The substrate is provided with a top layer 36 having smooth flat top surface 38. The pores that provide the porosity to the top layer and surface are much smaller than in the lower portion 37 of the substrate which functions as a reservoir for the substrate and which comprises the major portion of the substrate.

The reservoir 37 may be described as macroporous, meaning that the pores of the reservoir are visible without the aid of a microscope. The pore size range in the reservoir 37 is from 20̸ to 10̸0̸ µm (microns) and the pore volume will be between 35% to 65%. Normally a pore volume of 50̸% or greater is preferred and is necessary in some immunoassay applications. The pores in the top surface are preferably of a size that the top surface can be described as being microporous, which means that the pores in the top surface are so small that they can only be seen with the aid of a microscope.

To condition the device as described to be used in a testing process, microscopic particles, such as microspheres must be deposited on or in the top surface of the layer 36. The particles may also be trapped down in the top layer, by passing part way through the top layer before becoming entrapped. However, for the clearest indications of test results, it is preferred that the particles be trapped at the top surface. The particles may range in size from 0̸.1 µm (microns) to several µm (microns) in diameter. Particles larger than 5 µm (microns) may be employed, but smaller particles in the range of 0̸.1 to 5 µm (microns) are preferred, because smaller particles provide a greater concentration of surface area for the binding of a target substance to take place. Extremely small particles are not used because they would require correspondingly fine pores in the top surface in the substrate and the smaller the pore the top surface, the longer it takes the liquids in the immunoassay process to pass through the top layer into the reservoir. As a compromise between the competing concerns of obtaining a high concentration surface area and a rapid passing of the liquids through the top surface into the reservoir, microspheres of about 1 µm (micron) in size are most often selected. However, in some applications, such as when the sample to be tested contains low concentrations of a target antigen, a slower flow rate is desirable in order to give the antigen in the sample a longer time to react with the antibodies on the microspheres.

To deposit microspheres on the top surface of the substrate, the microspheres are suspended in an aqueous solution or a liquid which will wick readily into the substrate. A drop of the suspension containing the microspheres is placed on the top surface of the substrate and the liquid of the suspension wicks down into the substrate leaving the microspheres captured on or embedded in the surface of the substrate. The pore size of the top layer should be small enough so as to capture the microspheres on the top surface either by filtering action alone or in combination with a tendency of the microspheres to agglutinate and adhere to the material of the substrate. Usually, it is preferred that the pore size in the top surface should be as large as possible while still being effective to entrap the microspheres on or in the surface, unless a longer incubation time is needed in which case the pore size will be selected to provide the desired incubation time. In any event, the pore size of the top surface should be much smaller than the pore size of the reservoir, which as in the prior art system described with reference to Fig. 1, will function to draw liquids through the top layer by capillary action during the immunoassay test process. After the liquid of the suspension, has wicked down into the substrate with the microspheres being captured on the top surface 34 of the substrate, the liquid of the suspension is allowed to evaporate leaving the dried microspheres captured at on the surface of the substrate. Preferably the microspheres are immobilized on the substrate surface. This preferred immobilization is aided by agglutination of the microspheres and/or adherence of the microspheres to the material of the substrate.

The apparatus in this form, with the microspheres deposited on the surface of the substrate, is provided to the user who will carry out a testing process to test for the presence of a target substance. This testing process may be carried out substantially in the same manner as described above with respect to Fig. 1 and also as described in U.S. Patent No. 4,632,90̸1 to Valkiers et al issued December 30̸, 1986. Specifically, the microspheres deposited on the top surface of the substrate have bound or deposited on their surfaces antibodies, which are selected to be specific to the target antigen. A test sample to be tested for the presence of suspect antigen is poured on the top layer 36. The test sample is drawn through the top layer 36 relatively rapidly by the capillary action of the reservoir 37. If the suspect antigen is in the test sample, it will bind to the antibodies on the microspheres deposited on the top surface of the substrate. Following the application of the test sample to the substrate, a liquid containing labelled antibodies specific to the suspect antigen is poured on to the top layer and is drawn through the top layer into the reservoir 37 by capillary action. If the antigen has been extracted from the test sample and retained at the top surface of the substrate, the labelled antibodies will bind to the antigen and thus, remain at the top surface. As in the prior art process, the application of the labelled antibody to the top layer will usually be followed by a rinsing step to remove unbound labelled antibody from the top layer. This rinsing step is then followed by a step of causing the labelled antibody to exhibit its presence. In the case of an enzyme label this last step is carried out by the addition of a further solution of a color forming agent which reacts with the enzyme as the solution passes through the top layer 36.

As each liquid is passed through the top layer it is absorbed into the reservoir 37. Accordingly, the reservoir must be large enough to hold all of the volume of the test specimen, the labelled antibody solution, the rinse and the color forming solution. The apparatus of the invention may also be used in an immunoassay process, which tests for the presence of target antibodies in a test sample. In such a process, an antigen specific to the target antibodies is bound to or adsorbed on the surfaces of the microspheres or other small particles, and the target antibodies will bind to the antigen as the test sample is wicked through the top surface of the substrate. The apparatus of the invention may also be used in other diagnostic testing processes for detecting the presence of target substances in test samples other than immunoassay processes. To test for the presence of a target substance, a material which will adhere to the target substance is bound to or adsorbed on the surface of the small particles, or alternatively, the material of the small particles themselves is selected so that the target substance will adhere to the material of the small particles. Then when the test sample is wicked through the top surface of the top layer of the substrate, the target material will be extracted in or on the top surface of the substrate, where its presence is detected.

The one piece porous substrate with the top surface 38 having fine pores to trap the small particles on the surface can be manufactured by more than one process. In accordance with one embodiment, the porous substrate comprises sintered particles of synthetic resin with a top layer 36 being made from sintered particles smaller than the particles which are sintered together to form the reservoir. Suitable synthetic resins for the substrate are ultra high molecular weight polyethelene, high density polyethelene, or polyvinylidene fluoride, which is sold under the name Kynar. To facilitate forming the porous substrate by the methods described herein, the synthetic resin should be thermoplastic, but any synthetic resin which can be formed into an open celled macroporous substrate can be used. The material of the resin should be naturally hydrophilic or be capable of being rendered hydrophilic such as by means of a surfactant.

The smaller particles forming the top layer 36 will provide a pore size substantially smaller than that of the reservoir, but the pores would not be sufficiently small to entrap microspheres on the order of 5 µm (microns) in diameter or smaller on its surface. To achieve the fine pore size desired, in accordance with one embodiment, a microporous matrix is formed in the pores between the sintered particles of the top layers 36 by casting.

The microporous matrix is formed from a solution impregnated into the pores between the sintered particles of the layer 36 in a manner similar to the way that a microporous membrane is cast from a microporous membrane forming solution. The material of the microporous matrix should be hydrophilic or be capable of being rendered hydrophilic. Thus, the microporous matrix may be any material which can be cast from a solution within the pores at the top surface of the substrate and which material is or can be rendered hydrophilic. The material of the microporous matrix, for example, may be cellulose, polyvinylchloride, polyvinylidene fluoride, polycarbonate, nylon, or polysulphone.

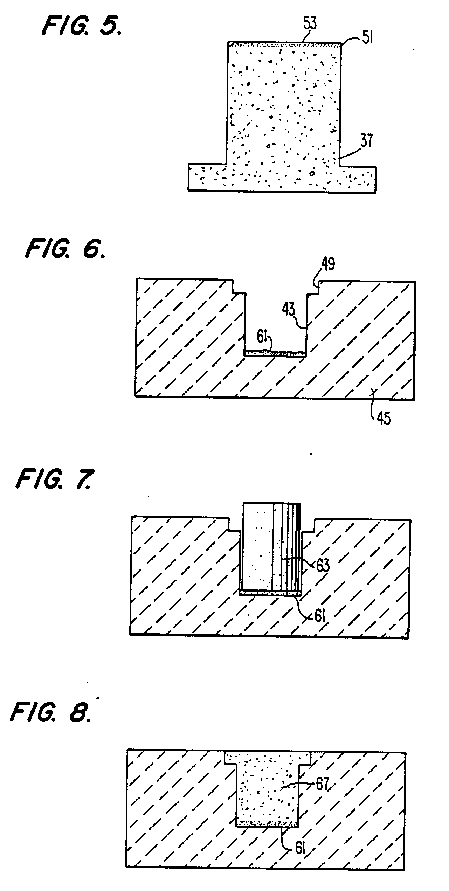

An example of a method of manufacturing the substrate of the invention is illustrated in Figs. 3 and 4. A layer of fine thermoplastic synthetic resin powder 41, e.g. ultra high molecular weight polyethelene, is positioned in the bottom of a cylindrical cavity 43 of a mold 45. The particles of the powder are of a size such that 60̸% of the particles or more will pass through a 150̸ mesh screen. Prior to positioning the layer of the fine powder 41 in the bottom of the mold cavity, a powdered surfactant 0̸.3% by weight is blended into the powder. An example of a surfactant which can be used is sodium-N-methyl-N-oleoyl taurate. The amount of fine powder forming the layer 41 is not critical, but it needs to be sufficient to completely cover the bottom of the mold cavity. After placing the fine powder in the bottom of the mold cavity, the mold cavity is vibrated to spread the powder evenly over the bottom surface and to insure that it completely covers the bottom surface of the mold cavity. The remainder of the cavity is then filled with thermoplastic synthetic resin powder 47 of a larger particle size, e.g. 10̸0̸ mesh powder, and the particles of which preferably are highly irregular in shape. The powder 47 may be the same resin as the layer 41, or it may be another resin which will sinter with the resin of layer 41. The use of irregularly shaped particles in the powder 47 keeps the particles from nesting close together and will result ultimately in a high void volume in the resulting porous structure, for example, 50̸% or greater. The powder 47 also has a powdered surfactant, 0̸.3% by weight, blended into it prior to the powder being placed in the mold. The surfactant may be the same as blended into the fine powder. The larger particle powder 47 fills the cavity 43 of the mold including a top section 49 of the cavity with an enlarged diameter. The mold is again vibrated to ensure that relatively uniform sized interstices are formed between the particles of powder 47. The mold is then heated to sinter the particles together. After the sintering, the mold is cooled and the slug comprising the sintered particles is removed from the mold. The sintered slug as shown in Fig. 5 at this point will have a top layer 51 which is formed of the fine sintered particles, and which will have a smooth top surface 53 formed by the bottom of the mold with an average pore size in the top layer and at the surface of between 5 and 10̸ microns. The average pore size formed of the larger particles which will ultimately comprise the reservoir 37 of the substrate will range from 20̸ to 30̸ µm (microns).

The sintered slug in the form immediately after molding could be used to entrap large microspheres in accordance with the invention. The average pore size in the top layer 53 will be between 5 and 10̸ µm (microns) and will entrap microspheres about twice the average pore size or larger at the surface and about 1/2 the average pore size in the top layer. However, as explained above, it is preferable to use microspheres 5 µm (microns) in diameter or less and to trap these microspheres at the surface of the substrate. After sintering the slug together as described above, a microporous matrix is cast within the pores of the substrate adjacent the top surface, while leaving the pores of the reservoir empty. Generally, the casting step is carried out by preparing a solution from which the microporous matrix is formed and impregnating the pores adjacent to the smooth top surface of the slug with the solution. A phase inversion is caused to occur in the impregnated solution and the solid material which becomes the microporous matrix comes out of solution within the pores of the slug adjacent to the top surface of the slug. In this manner, a smooth microporous surface which will entrap the small microspheres is provided.

Examples of solutions for forming the microporous matrix and methods of casting the microporous matrix in the pores are described below:
40̸0̸ grams of n-butyl alcohol (butanol) is poured into 1 gallon mixing bucket of a commercial mixer capable of running at 250̸0̸ rpm. With the mixer running at low speed, 10̸0̸0̸, 16 grams of cellulose acetate are slowly added to the butanol. After the cellulose has been added, the mixer is run at 250̸0̸ rpm for one minute the end of which the cellulose acetate should be dissolved.

132.8 grams of nitrocellulose, which is wet with 30̸% by weight isopropopal alcohol and which has a viscosity rating between 70̸ and 80̸ seconds (ASTM D 1343-56) is added to the mixing bucket and the mixer is run at 250̸0̸ rpm. for two minutes to dissolve the nitrocellulose.

10̸79 grams of acetone is poured into the mixing bucket and the mixer is operated at high speed for fifteen minutes. At the end of this mixing step, all of the solids will be dissolved in the mixture.

732 grams of butanol is then poured into the mixer and the mixer operated at high speed for two minutes.

Then with the mixer running at 10̸0̸0̸ rpm. 10̸0̸ grams of filtered pure water is dribbled slowly into the solution.

With the mixing speed still running at 10̸0̸0̸ rpm, 24 grams of colorent grade titanium dioxide (TiO₂) are mixed into the solution, after which the mixer is run at 320̸0̸ rpm for fifteen minutes so that the titanium dioxide is thoroughly mixed and in suspension in the liquid of the solution.

The mixture is then poured into a closed container which has a small space above the top of the solution. The mixture will contain air bubbles as a result of the mixing process. The solution is allowed to stand for several hours, during which time the bubbles in the solution will rise to the surface and escape. During the removal of the bubbles, the temperature of solution is kept above 26.7°C (80̸ degrees F). The finished mixture, after removal of the air bubbles, at 32.2°C (90̸ degrees F). should have a viscosity of 40̸0̸ to 80̸0̸ MPa.s (centipoise ; cps) and is ready to be impregnated into the fine pores adjacent to the smooth top surface of the porous plastic slug. In the casting process for this example, a phase inversion is caused to occur by the evaporation of the solvents leaving behind the microporous matrix as a residue within the pores of the substrate. In order to achieve consistent repeatable results in the porosity of the microporous matrix, the casting process should be performed in a controlled environment. Preferably the relative humidity should be kept between 70̸ and 80̸ percent and the ambient temperature should be kept between 25.6 and 27.8°C (78 and 82 degrees F).

In the casting process the solution is heated to a temperature of between 43.3 and 46.1°C (110̸ and 115 degrees F), which will drop the viscosity of the solution to below 50̸0̸ MPa.s (centipoise). A plurality of the slugs formed as described above are oriented in a rack so that the smooth top surface of the slug protrudes below the bottom of the rack. The heated casting solution is poured into a shallow trough and the rack is placed over the trough so that the smooth surface of the slugs are immersed approximately 3.2 mm (1/8 of an inch) deep into the solution at which time the mixture in the trough will impregnate the pores at the top surface. The tops of the slugs are allowed to remain in the solution for approximately 1 second, whereupon the rack is lifted straight up and then turned over slowly enough for the excess fluid to run off. The rack with the smooth top surfaces facing upwardly with the solution now impregnated in the fine pores adjacent to the top surface is placed under an exhaust hood containing an exhaust fan which is operated to draw air over the impregnated slugs. With a hood having a lower inlet end of 660 x 965 mm (26 inches by 38 inches), the air is exhausted from the hood at a rate of 2.1 m³ (75 cubic feet) per minute to achieve a desired rate of air flow over the impregnated slugs. These steps are all performed while maintaining the controlled humidity and temperature as described above. The impregnated slugs are allowed to sit under the exhaust hood while air is being drawn over the slugs for sufficient time for the solvents to evaporate and the microporous matrix to form and harden in the slug. This time interval will be at least 1 1/2 hours. The parts are then removed from the controlled environment and approximately 0̸.5 milliliters of a dilute surfactant solution is applied to the smooth top surface of each substrate. For example, the solution may contain 1% by weight of Varion™, 10̸% by weight isopropyl alcohol, and 89% by weight filtered water. Varion is a surfactant available from Sherex Corporation. After application of the surfactant solution, the parts are then placed in a moving air stream to dry them, whereupon the manufacture of the substrates is completed.

The above example will result in a substrate which is microporous at the top surface 38 and macroporous in the reservoir 37 with a high void volume over 50̸% with the reservoir pores in capillary communication with the micropores at the smooth top surface of the substrate. The substrates prepared in accordance with this example will consistently trap on their top surfaces polystyrene microspheres as small as 1.0̸ µm (microns) in diameter.

By changing the parameters of the solution from which the microporous matrix is cast the porosity of the microporous matrix can be varied so as to entrap other sizes of microspheres. If the percentages of the solids added to the solution is increased, the pore size of the microporous matrix will be reduced. If the molecular weight of the nitrocellulose is increased, the pore size will be reduced. The molecular weight of nitrocellulose is usually specified by the vendor as a viscosity rating per ASTM D 1343-56.

In an alternative specific example, the initial amounts of the liquids used in the solution, butanol, acetone, and water are reduced by 40̸% from the above described specific examples and 132.8 grams of nitrocellulose with a viscosity rating between 140̸ and 180̸ seconds is substituted for nitrocellulose of the above described example. The resulting solution will have a viscosity of 350̸0̸-550̸0̸ MPa.s (cps). The solution is heated to a temperature of between 51.7 and 57.2°C (125 and 135 degrees F), which will drop the viscosity below 30̸0̸0̸ MPa.s (cps), and the solution is impregnated into the top surface of the slugs by dipping as described above. With these changes, and otherwise following the specific example as described above, the pore size of the resulting microporous matrix will be reduced so that the top surface will trap microspheres down to 0̸.3 µm (microns) in size.

Instead of dipping the slugs into the microporous matrix forming solution, the solution may be dispensed onto the smooth top surface of the slugs by means of a dispensing gun. As in the dipping process, the entire operation of applying the solution to the top surfaces of the slugs should be performed in a controlled environment. The relative humidity of the environment should be kept between 70̸ and 80̸ percent and the temperature should be kept between 25.6 and 27.8°C (78 and 82 degrees Fahrenheit). The membrane solution of the first described example is heated to a temperature of 43.3 to 46.1°C (110̸ to 115 degrees Fahrenheit) to drop the viscosity of the solution below 50̸0̸ MPa.s (cps). The heated solution is put into a heated fluid dispensing gun and by means of the gun, and a measured amount of the solution is dispensed from the gun onto each slug top surface. The amount of the solution dispensed is selected to be a little more than enough to cover the top surface. The microporous matrix is then allowed to form from the solution within the pores of the top surface under an exhaust hood as described before. After hardening of the microporous matrix, the dilute surfactant solution is applied as described before. The resulting structures will consistently trap on their top surfaces 1.0̸ µm (micron polystyrene microspheres).

Another way of achieving a microporous matrix which will entrap 0̸.3 µm (micron) microspheres is to dispense a second measured amount of the solution into the top surface of each slug from the gun. The second measured amount is preferably dispensed onto the top surface at least a few minutes after dispensing the first measured amount so that the microporous matrix from the first measured amount has begun to form. The parts are then dried under the drying hood in the same manner as described above again for 1.5 hours. This repeated application of the microporous matrix forming solution Will decrease the pore size in the matrix in a top surface so that the top surface will entrap 0̸.3 µm (micron) microspheres.

The above-described examples will entrap microspheres of the size described with no proteins bonded to the surface of the microspheres and will entrap microspheres with antibodies on the microspheres of the same size if there is no chemical attraction between the antibodies and the material of the microporous matrix. However, if there is a chemical attraction between the antibodies on the surface of the microsphere and the material of the microporous matrix, then the structure will entrap still, smaller microspheres. For example, in the example which will entrap one µm (micron) microsphere, if there is a chemical attraction between the antibodies and the cellulose material of the microporous matrix, then microspheres down to 0̸.5 µm (microns) can be entrapped at the top surface of the structure.

In an alternative embodiment, the substrate is made without the use of a microporous matrix cast within the pores of the top layer. In this embodiment, a very fine thermoplastic synthetic resin powder, e.g. ultra high molecular weight polyethylene powder, is used to form the top layer of the slug, specifically, a layer having at least 90̸% of the particles thereof small enough to pass through 20̸0̸ mesh (U.S. sieve) screen. A powder in which the particles will pass through a 20̸0̸ mesh screen is referred to as being "minus 20̸0̸ mesh". Thus the powder employed is 90̸% minus 20̸0̸ mesh. This fine powder is produced by taking commercially available synthetic resin powder, and vibrating it over a 20̸0̸ mesh screen. The particles that pass through the screen are collected to be used as the top layer 35 of the substrate.

The fine powder produced as described above is blended with a 0̸.3% by weight of surfactant, which may for example be sodium-N-methyl-N-oleoyl taurate. The mixture of the powder and the surfactant is dispensed into the cavity 43 of the mold 45 as shown in Fig. 6. The mold is vibrated to spread the fine powder evenly over the bottom of the cavity 43 into a layer 61. The amount of powder dispensed in the cavity is not critical but it should be sufficient to completely cover the bottom of the mold cavity. After the powder has been spread evenly over the bottom of the mold, a cylindrical pin 63 having a diameter slightly smaller than the diameter of the lower part of the cavity 43 is inserted into the cavity and pressed against the layer 61 of fine powder with a force of 34474 to 103422 kPa (50̸0̸0̸ to 150̸0̸0̸ pounds per square inch) to compress the powder is shown in Fig. 7. This compression reduces the size of the interstices between the particles of the fine powder and accordingly will reduce the pore size in the top layer 35 of the substrate being produced. The greater the compressive force applied to the layer 61, the smaller will be pore size in the top layer 35 of the resulting substrate. A force of 75843 kPa (11,0̸0̸0̸ pounds per square inch) will produce an average pore size diameter in the top layer 35 of the resultant substrate of about 3 microns. After the layer 61 has been compressed, the remainder of the mold cavity 63 is filled with larger particle synthetic resin powder 67, e.g. 150̸ mesh ultra-high polyethlene powder, as shown in Fig. 8. The powder 67 will also have a surfactant, 0̸.3% by weight, blended therein. The surfactant may be the same as in the fine powder of the layer 61. The mold is then again vibrated for about 20̸ seconds to achieve a more uniform spacing between the particles 67. This vibration will not substantially affect the compressed layer 61. The mold is then heated to sinter the particles of the layer 61 and the particles of the powder 67 together and the two layers together at their interface. The mold is then cooled and the finished substrate is dropped out of the mold. The resulting substrate will have a top layer 35 which is microporous and a reservoir 37 which is macroporous and which is in capillary communication with the microporous layer. The microporous top layer will entrap microspheres of about 5 µm (microns) on the top surface if there is no chemical bonding between antibodies in the microspheres and the material of the top layer 35. If chemical bonding occurs, the top surface and layer will entrap smaller microspheres. The top layer will also trap microspheres down to a µm (micron) in diameter distributed through the top layer.

In the immunoassay apparatus described above, a one piece porous substrate performs the function of entrapping microspheres at the surface of the substrate or in the top layer while at the same time acting as a reservoir to accumulate the liquids that are passed through the upper surface of the substrate in the immunoassay process. Thus, the problems of assembly of components of the substrate and problems of gaps between components of the substrate are completely avoided.

## Claims

1. A diagnostic apparatus comprising an open-celled porous substrate having a top layer (36) and a lower reservoir portion (37), said top layer being microporous and functioning to transmit aqueous liquid from the top surface (38) of said top layer to said reservoir portion by capillary action, said reservoir portion being macroporous and being in capillary communication with the micropores of said top surface, said substrate having microparticles deposited on said surface, said microparticles having antigens or antibodies bound or absorbed on the surface thereof characterized in that said top layer and said lower reservoir portion are constructed as one piece.

2. A diagnostic apparatus as recited in claim 1, wherein said substrate is contained within a container (33), said container having a top opening exposing said top surface.

3. A diagnostic apparatus as recited in claim 1, wherein said microparticles are microspheres.

4. A diagnostic apparatus as recited in claim 1, wherein said substrate comprises sintered particles of synthetic resin and a microporous matrix cast in the pores defined by the sintered particles of said substrate at said top surface.

5. A diagnostic apparatus as recited in claim 4, wherein said sintered particles comprise a layer of particles adjacent to said top surface which are smaller than the remainder of said particles.

6. A diagnostic apparatus as recited in claim 1, wherein said top layer has micropores of a size to entrap microspheres in or on said top layer when applied to said top layer in a liquid suspension with the liquid passing through said top layer into said reservoir portion.

7. A diagnostic apparatus as recited in claim 1, wherein said substrate comprises sintered particles of synthetic resin and wherein said top layer comprises sintered particles which are smaller than the sintered particles in said reservoir.

8. A diagnostic apparatus as recited in claim 8, wherein a microporous matrix is cast in the pores defined by the sintered particles of said top layer adjacent to the top surface thereof.

9. A diagnostic apparatus as recited in claim 8, wherein said top layer of sintered particles is formed by distributing said smaller particles in a layer and applying a compressive force to said layer to reduce the size of the interstices between said particles and then sintering said particles together.

## Patentansprüche

1. Diagnosevorrichtung, aufweisend ein offenzelliges, poröses Substrat mit einer oberen Schicht (36) und einem unteren Reservoirteil (37), wobei die obere Schicht mikroporös ist und dahingehend wirkt, wässrige Flüssigkeit von der Oberfläche (38) der oberen Schicht in den Reservoirteil durch Kapilarwirkung zu übertragen, wobei der Reservoirteil makroporös ist und in kapilarer Verbindung mit den Mikroporen der Oberfläche steht, wobei das Substrat Mikropartikel aufweist, die auf der Oberfläche abgelagert sind, wobei die Mikropartikel Antigene oder Antikörper aufweisen, die gebunden oder an deren Oberfläche absorbiert sind, dadurch gekennzeichnet, daß die obere Schicht und der untere Reservoirteil in einem Stück ausgebildet sind.

2. Diagnosevorrichtung nach Anspruch 1, dadurch gekennzeichnet, daß das Substrat innerhalb eines Behälters (33) enthalten ist, wobei der Behälter eine obere Öffnung aufweist, die die Oberfläche freilegt.

3. Diagnosevorrichtung nach Anspruch 1, dadurch gekennzeichnet, daß die Mikropartikel mikrosphärische Teilchen sind.

4. Diagnosevorrichtung nach Anspruch 1, dadurch gekennzeichnet, daß das Substrat gesinterte Partikel aus synthetischem Harz ist und eine mikroporöse Matrix aufweist, die in die Poren eingegossen ist, die durch die gesinterten Partikel des Substrats an der oberen Fläche gebildet sind.

5. Diagnosevorrichtung nach Anspruch 4, dadurch gekennzeichnet, daß die gesinterten Partikel eine Schicht aus Partikeln aufweisen, die in der Nähe der Oberfläche angeordnet sind, welche kleiner als der verbleibende Teil der Partikel sind.

6. Diagnosevorrichtung nach Anspruch 1, dadurch gekennzeichnet, daß die obere Schicht Mikroporen einer Größe hat, um die mikrosphärischen Teilchen in oder an der Oberfläche zu umschließen, wenn auf dieser oberen Fläche eine Flüssigsuspension mit der Flüssigkeit aufgebracht wird, die durch die obere Schicht hindurch in den Reservoirteil hindurchgeht.

7. Diagnosevorrichtung nach Anspruch 1, dadurch gekennzeichnet, daß das Substrat gesinterte Partikel aus synthetischem Harz aufweist und daß die obere Schicht gesinterte Partikel aufweist, die kleiner als die gesinterten Partikel in dem Reservoir sind.

8. Diagnosevorrichtung nach Anspruch 7, dadurch gekennzeichnet, daß eine mikroporöse Matrix in die Poren eingegossen ist, die durch die gesinterten Partikel der oberen Schicht in der Nähe der oberen Oberfläche gebildet sind.

9. Diagnosevorrichtung nach Anspruch 8, dadurch gekennzeichnet, daß die obere Schicht der gesinterten Partikel durch Verteilen der kleineren Partikel in eine Schicht und durch Anlegen einer Druckkraft an die Schicht gebildet ist, um die Größe der Zwischenräume zwischen den Partikeln zu verringern und sodann die Partikel zusammenzusintern.

## Revendications

1. Appareil de diagnostic comprenant un substrat poreux à alvéoles ouverts présentant une couche supérieure (36) et une partie inférieure formant réservoir (37), ladite couche supérieure étant microporeuse et fonctionnant pour laisser passer par capillarité un liquide aqueux depuis la surface supérieure (38) de ladite couche supérieure, vers ladite partie formant réservoir, ladite partie formant réservoir étant macroporeuse et étant en communication capillaire avec les micropores de ladite surface supérieure, ledit substrat présentant des microparticules déposées sur ladite surface, lesdites microparticules comportant des antigènes ou anticorps liés ou absorbés à la surface de celles-ci,
caractérisé en ce que ladite couche supérieure et ladite partie inférieure formant réservoir sont conçues d'un seul tenant.

2. Appareil de diagnostic selon la revendication 1, dans lequel ledit substrat est contenu à l'intérieur d'un conteneur (33), ledit conteneur présentant une ouverture supérieure par laquelle est exposée ladite surface supérieure.

3. Appareil de diagnostic selon la revendication 1, dans lequel lesdites microparticules sont des microsphères.

4. Appareil de diagnostic selon la revendication 1, dans lequel ledit substrat comprend des particules frittées, en résine synthétique, et une matrice microporeuse, moulée dans les pores définis par les particules frittées dudit substrat, à ladite surface supérieure.

5. Appareil de diagnostic selon la revendication 4, dans lequel lesdites particules frittées comprennent une couche de particules adjacentes à ladite surface supérieure, et qui sont plus petites que le reste desdites particules.

6. Appareil de diagnostic selon la revendication 1, dans lequel ladite couche supérieure présente des micropores d'une taille permettant l'inclusion de microsphères dans ou sur ladite couche supérieure, lors de leur application sur ladite couche supérieure dans une suspension liquide, le liquide passant à travers ladite couche supérieure pour aller dans ladite partie formant réservoir.

7. Appareil de diagnostic selon la revendication 1, dans lequel ledit substrat comprend des particules frittées, en résine synthétique, et dans lequel ladite couche supérieure comprend des particules frittées qui sont plus petites que les particules frittées qui sont dans ledit réservoir.

8. Appareil de diagnostic selon la revendication 7, dans lequel une matrice microporeuse est moulée dans les pores définis par les particules frittées de ladite couche supérieure, adjacentes à ladite surface supérieure de celle-ci.

9. Appareil de diagnostic selon la revendication 8, dans lequel ladite couche supérieure de particules frittées est constituée en répartissant lesdites particules plus petites en une couche et en appliquant une force de compression à ladite couche pour réduire la taille des interstices entre lesdites particules, puis en frittant ensemble lesdites particules.
